(19) Europäisches Patentamt
European Patent Office
Office européen des brevets

(11) Publication number: **0 299 667 A1**

(12) # EUROPEAN PATENT APPLICATION

(21) Application number: 88306136.8

(51) Int. Cl.4: **A61B 5/04**

(22) Date of filing: 06.07.88

(30) Priority: 10.07.87 JP 105307/87
10.07.87 JP 105308/87
29.07.87 JP 115086/87
11.12.87 JP 187670/87

(43) Date of publication of application:
18.01.89 Bulletin 89/03

(84) Designated Contracting States:
DE FR GB

(71) Applicant: KOIKE SEIKI KABUSHIKI KAISHA
5-10, Minami-Azabu 1-chome
Minato-ku Tokyo(JP)

(72) Inventor: Koike, Hiroshi
5-10, Minami-Azabu 1-chome
Minato-ku Tokyo(JP)
Inventor: Suzuki, Shoji
2-6-802, Iwato-minami 1-chome
Komae-shi Tokyo(JP)

(74) Representative: Bubb, Antony John Allen et al
GEE & CO. Chancery House Chancery Lane
London WC2A 1QU(GB)

(54) **Portable electrocardiograph.**

(57) An electrocardiograph capable of recording event data representing specific action or paroxysmal symptoms of a patient along with electrocardiogram data detected continuously from the patient over a long time has operating event-inputting keys (8) which indicate on their top surfaces the specific actions and paroxysmal symptoms in the form of a figure and/or character. The event data can thus be entered easily by the patient for recording together with the electrocardiogram data recorded at the time of the event.

FIG. 4

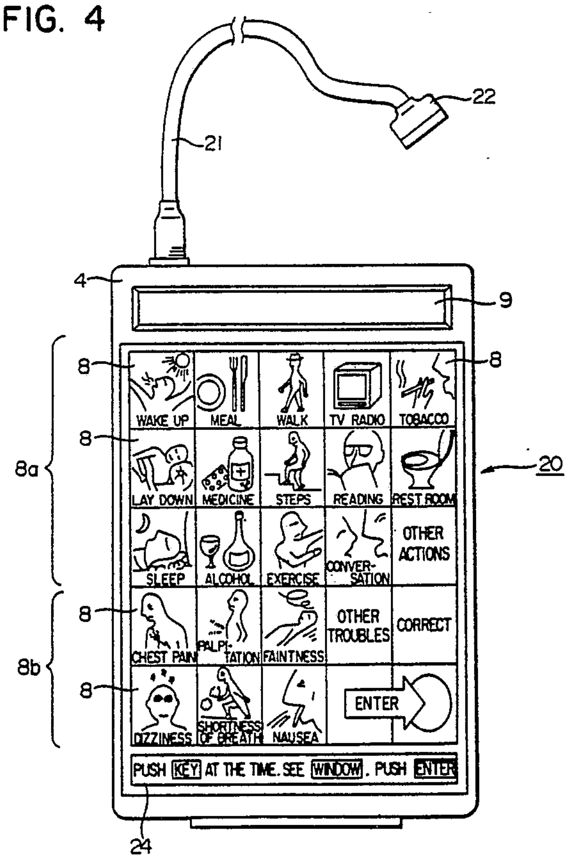


EP 0 299 667 A1

Xerox Copy Centre

# PORTABLE ELECTROCARDIOGRAPH

This invention relates to a portable electrocardiograph for measuring and recording, over a long time, an electrocardiogram of a patient suffering from heart disease or the like. More particularly, this invention relates to a portable electrocardiograph capable of recording event data representing the specific actions or paroxysmal symptoms of a patient at any time together with an electrocardiogram which is continuously detected over a long time and outputting the detected electrocardiogram for diagnostic purposes.

Analytical diagnostics by use of electrocardiograms have been generally applied for making a diagnosis of for example cardiopahty. As a portable electrocardiograph for detecting and recording an electrocardiogram over a long time, there has been widely used the so-called Holter's electrocardiograph of a portable type with a built-in cassette tape recorder. The electrocardiograph of this type has a function of recording the electrocardiogram continuously detected from a patient for twenty-four hours on a cassette tape loaded in the built-in tape recorder and outputting the detected electrocadiogram to an external computer or the like for post diagnostic purposes. In case that the electrocardiogram is continuously recorded by such a conventional electro cardiograph over a whole day and night, a task of writing a diary is assigned to the patient. Namely, it is necessary to precisely record a day's personal history, e.g. specific actions and paroxysmal subjective symptoms, of the patient in a recording paper with time data in order to weigh the detected electrocardiogram with the condition of the patient for making the post diagnosis. The task of recording the patient's events such as subjective symptoms becomes more onerous than might be expected and is wanting in precisenss in time.

Furthermore, the work of agreeing waveforms of the detected electrocardiogram with the event data inputted by the patient for making a diagnosis turns out to be troublesome.

In view of the aforesaid drawbacks of conventional portable electrocardiographs, it is an object of the present invention to provide an improved portable electrocardiograph capable of automatically recording an electrocardiogram without interruption over a long time on a recording medium, and enabling event data representing specific actions, paroxysmal subjective symptoms and so on of a patient to be recorded with time data and simultaneously displayed for confirmation when inputting the aforesaid event data.

Another object of the present invention is to provide a portable electrocardiograph which enables the electrocardiogram detected successively to be monitored in real time.

To attain the object described above according to this invention, there is provided a portable electrocardiograph having a function of continuously recording an electrocardiogram derived from electrocardiography electrodes attached to a patient in a built-in recording medium, which comprises a plurality of event-inputting keys for inputting event data representing specific actions or paroxysmal subjective symptoms of the patient, each key having a keytop indexing one of the specific actions and paroxysmal symptoms in the form of a figure and/or character, and an event display on which the event data inputted by operating the event-inputting keys are displayed in the form of a figure and/or character.

The electrocardiogram which is detected as electric signal waveforms from the electrocardiography electrodes attached to the chest of the patient is recorded by the built-in recording medium and, at the same time, the event information representing one of the specific actions and paroxysmal symptoms of the patient by operating the corresponding event-inputting key when, for instance, the patient takes exercise or has a paroxysm. Thus, the specific actions and/or paroxysmal symptoms of the patient can be observed for making a post diagnosis with reference to the detected electrocardiogram displayed along with the inputted event information on one monitor screen. Since the event information which is inputted by operating the event-inputting key is displayed on the event display in the form of a figure and/or character, it can be recorded correctly and readily. As a recording medium, a semiconductor memory such as a randam access memory (RAM) or a magnetic recording tape can be used.

By providing the electrocardiograph with a clock function, time data can be recorded along with the detected electrocardiograms when the event data are inputted, and time indication can be fulfilled at all times. The electrocardiogram recorded by the built-in recording medium is generally outputted to an external computer system so as to be displayed on a display device for making a diagnosis. By providing a monitor screen on the present electrocardiograph, some waveforms of the detected electrocardiogram can be simply displayed in real time for confirmation.

One way of carrying out the invention is described in detail below with reference to drawings which illustrate only one specific embodiment, in which:-

Figure 1 is a perspective view schematically showing one preferred embodiment of the portable electrocardiograph according to this invention.

Figure 2 is a block circuit diagram of the same.

Figure 3 is an explanatory plan view of the same, illustrating one example of arrangement of the event-inputting keys.

Figures 4 and 5 are an explanatory plan view of the separated keyboard and a perspective view, which schematically illustrate another embodiment of this invention.

The portable electrocardiograph according to this invention has a function of recording event data such as specific actions and paroxysmal subjective symptoms of a person to be diagnosed (patient) along with an electrocardiogram (ECG) detected from the patient on a built-in recording medium. The first embodiment of the electrocardiograph of the invention is shown in Figures 1 to 3.

In the drawings, reference numeral 1 denotes an ECG body of the portable electrocardiograph of light weight and small size as a Holter's electrocardiograph. A connector 2 serves to detachably connect ECG electrodes 3 to the body 1. Inside a casing 4 of the ECG body 1, a processing circuit for detecting and recording the ECG data from the patient and enabling the aforesaid event data to be inputted at any time along with the ECG data. As shown in Figure 2, this circuit basically comprises an analogue-to-digital (A/D) converter 5 for converting an ECG waveform signal which is detected by the ECG electrodes 3 attached to the patient's chest to a digital signal, a central processing unit (CPU) 6 for performing arithmetic on variable data and controlling component circuits, a recording unit (recorder) 7 having a recording medium on which time-serial data signals from the A/D converter 5 via the CPU 6, and a clock means 13. In this embodiment, as the recording medium, a semiconductor integrated memory element such as a randam access memory (RAM) may be utilized. In this case, the RAM having a storage capacity to satisfy the required time for recording the detected ECG data should be used.

Furthermore, the electrocardiograph in this embodiment is provided on the upper surface of the casing 4 with a plurality of event-inputting keys 8. On the keytops of these keys 8, figures and/or characters representing the specific actions and paroxysmal subjective symptoms or the condition of a patient are indexed as illustrated in Figure 3. The kays 8 may be arranged as classified into two blocks, an action block 8a indicating daily life events of the patient (e.g. waking, lying-down, sleeping, meal, medication, drinking, walking, stair-ascension, exercise, audiovisual appreciation, reading, conversation, smoking, and easing nature), and a symptomatic block 8b indicating paroxysmal symptoms (e.g. chest pain, vertigo, palpitation, short breath, faint, and nausea). With this key arrangement, the work of inputting event data becomes easy. Additionally, a correction or clear key 8c, a decision key 8d, and various function keys are provided. Furthermore, for adjusting the time of the built-in clock means, index numerals may be printed on the keytops of some keys 8.

The key structure adopted in this embodiment as illustrated is composed of one flexible keytop sheet covering the plurality of event keys 8, on which the aforementioned event figures and/or characters are printed at the positions opposing the respective event keys. Of course, each key may be possessed of an individual keytop as separated from the other keys.

On the upper portion of the casing 4, there is formed an event display 9 in confirmation of the event data inputted by operating the event-inputting keys 8. The event display 9 has a function of manifesting the inputted event data in the form of a character and/or figure. The event display 9 may preferably be composed of a display unit with small power dissipation such as a liquid crystal display element (LCD). Also, the time controlled by the clock means 13 may be indicated on the display 9 at all times.

The control unit for controlling and operating the aforementioned keys 8 and display 9 effects to cause the CPU 6 upon reception of a key code signal which is generated by a key code generator 10 when the key is operated to drive the display 9 to thereby display the corresponding figure and/or character representing the event data.

By 11 is denoted a data-outputting connector disposed on one side of the casing 4. To the connector 11, an external computer C and a printer P can be connected so as to output the ECG data. Reference numeral 12 denotes a power switch.

In the case of detecting the ECG data from the patient by the electrocardiograph 1 having the aforementioned basic structure, the patient may lead general everyday life with the present electrocardiograph having the ECG electrodes 3 attached to his chest. The ECG data detected by the ECG electrodes 3 are fed to the CPU 6 via the A/D converter 5 and recorded as time-serial data in the recording unit 7.

In the course of detecting and recording the ECG data, at the time one relevant key in the action key block 8a is pressed, the event information is displayed on the event display 9. Then, when the decision key 8d is pressed upon confirmation of the event information displayed on the event display 9, the event signal representing the action of the patient is recorded in the recording unit 7 along with the ECG data noted above.

When the patient has a paroxysm, the event signal repressnting the paroxysmal symptoms of the patient is recorded in the recording unit 7 by pressing the relevant key in the symptomatic key block 8b in the same manner as above. In the case where the ECG data detected and recorded by the present electrocardiograph 1 are applied for a diagnosis, the ECG data are transferred to the external computer C connected electrically to the connector 11 disposed on the casing 4.

The ECG data transferred from the electrocardiograph 1 to the computer C are stored in a memory device in the computer and analyzed by use of an electrocardiography program, and thereafter, the analytic results thus obtained are displayed on a display device D. The ECG waveform pattern is displayed serially scrolling on the display device D. When displaying the ECG waveform which is obtained at the time that the specific event key 8 is pressed, the corresponding event information is simultaneously displayed on the same display screen. Besides, the ECG waveform can be printed out by the printer P as occasion arises.

Generally, the ECG data such as the ECG waveform and numeral data are sent in a lump to the printer P via the computer C. However, the ECG data may be outputted directly to the printer P as shown by the dotted line in Figure 2 so as to obtain a hard copy of the ECG waveform.

The ECG data detected by the ECG electrodes from the patient are recorded in the recording unit 7 in terms of time-serial signals along with the time data derived from the built-in clock means 13. Therefore, any portion of the ECG waveform can be diagnostically recognized by the time simultaneously displayed on the display screen.

Another embodiment illustrated in Figures 4 and 5 has a keyboard 20 separated from the electrocardiograph body 1. The keyboard 20 comprises the aforementioned event-inputting keys 8 and is electrically connected in a detachable manner to the body 1 by a connection cable 21 provided on its leading end with a plug 22. The plug 22 is detachably coupled with a socket 23 disposed on one side of the body 1 as illustrated in Figure 5. Two group of the event-inputting keys, 8a and 8b, are enclosed with the respective line frame so as to facilitate the operations of the keys. Moreover, guidance messages 24 may be printed in a blank space on the keyboard 20 as illustrated in Figure 4.

The electrocardiograph shown in Figure 5 contains a tape recorder 25 using a magnetic recording tape as a recording medium. As the recording tape, a general-purpose compact cassette tape or micro cassette tape may be used. Though the ECG data stored in the RAM in the foregoing embodiment are given as digital signals, those in this embodiment may be in an analogue form because the tape recorder can record an analogue signal.

In this embodiment, a monitor screen 26 is provided on the side of the tape recorder 25. It is possible to display on the monitor screen 26 the ECG waveform while detecting in real time and reproduce the ECG data recorded by the tape recorder 25.

The ECG waveform is displayed on the monitor screen 26 scrolling to, for example, right along with the event information. By monitoring the ECG data on the monitor screen 26, a check can be easily made on whether the detection of the ECG data is carried out normally and the ECG electrodes are set on the patient's chest without fail.

The monitor screen 26 may be preferably composed of a liquid crystal display element (LCD). The event information as mentioned above may be displayed on the monitor screen 26. In this case, the display 9 used in the foregoing embodiment can be removed.

As is apparent from the above, according to the electrocardiograph of this invention, in which a plurality of event-inputting keys indexing, on their keytops, specific actions and paroxysmal symptoms of a patient in the form of a figure and/or character, ECG data detected from the patient can be automatically recorded continuously on a built-in recording medium over a long time and event data such as the specific actions and paroxysmal symptoms of the patient can be easily recorded with the detected ECG data for a post diagnosis. Besides, since the present electrocardiograph is provided with an event display for displaying event information inputted by operating the event keys, manual input to the electrocardiograph can be correctly carried out with ease. As the electrocardiograph of this invention has a clock function, the event data representing the specific action or paroxysmal symptoms of the patient can be automatically recorded with time data, thereby to enable a precise post diagnosis to be made easily.

What is more, by separating a keyboard having the event keys from the electrocardiograph body, the event data can be readily inputted remotely to the electrocardiograph, and the device can be handled with ease. By providing the present electrocardiograph with a monitor screen capable of displaying the detected ECG waveform in real time, a check can be easily made for the ECG data while detecting and the ECG electrodes attached to the patient. Thus, this invention provides an improved Holter's electrocardiograph which can be used quite advantageously and effectively such as when heart disease is diagnosed.

## Claims

1. A portable electrocardiograph comprising an electrocardiograph body (1) containing a recording medium (7,25) for continuously recording analogue or digital electrocardiogram data which are detected by electrocardiography electrodes (3) attached to a patient, a plurality of event-inputting keys (8) indicating specific actions and paroxysmal symptoms of the patient in the form of a figure and/or character which serve to output event data repesenting the specific action or paroxysmal symptoms so as to record the event data on said recording medium (7,25), and an event display (9) for displaying the event data inputting by operating said event-inputting keys (8) in the form of a figure and/or character.

2. A portable electrocardiograph according to claim1, further comprising a clock means (13) for recording time data along with the electrocardiogram data and the event data on said recording medium (7,25) when said event-inputting key (8) is operated.

3. A portable electrocardiograph according to any of claims 1 and 2, wherein said recording medium (7,25) is a semiconductor memory.

4. A portable electrocardiograph according to any of claims 1 and 2, wherein said recording medium (7,25) is a magnetic recording tape.

5. A portable electrocardiograph according to any of claims 1 to 4, comprising a monitor screen (26) for displaying the detected electrocardiogram data in a waveform.

6. A portable electrocardiograph according to any of claims 1 to 5, wherein said event-inputting keys (8) are integrally formed on said electrocardiograph body (1).

7. A portable electrocardiograph according to any of claims 1 to 6, wherein said event-inputting keys (8) are formed on a keyboard (20) which is separated from and electrically connected to said body (1).

FIG. 1

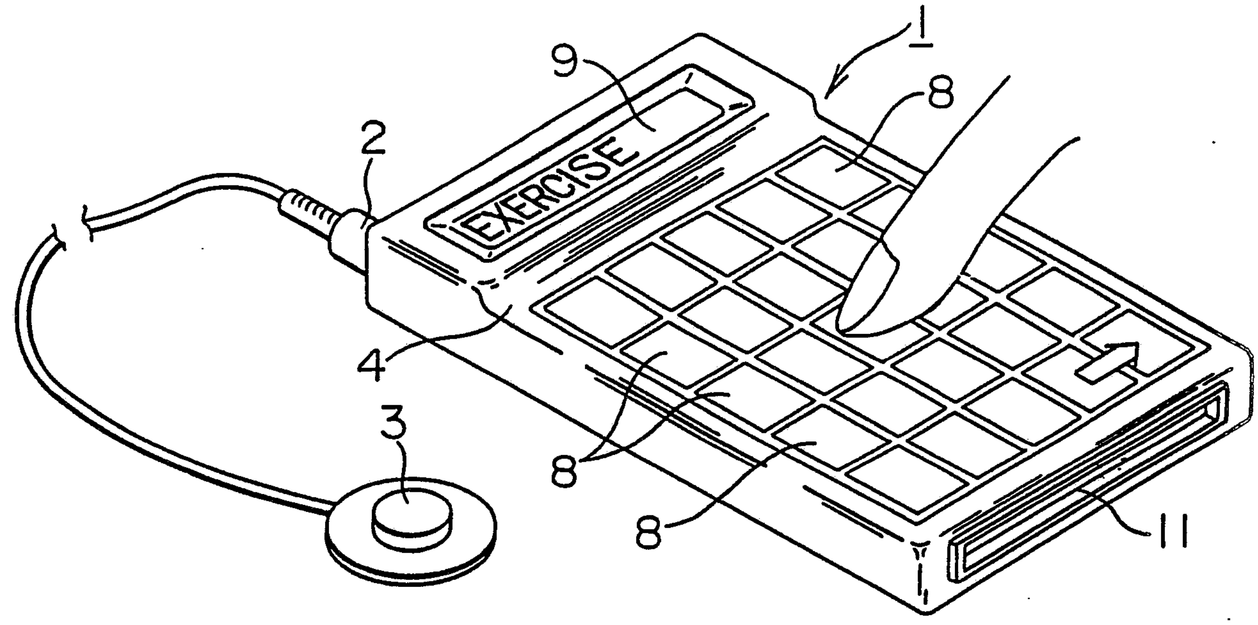
1
9
8
2
EXERCISE
4
3
8
8
11

FIG. 2

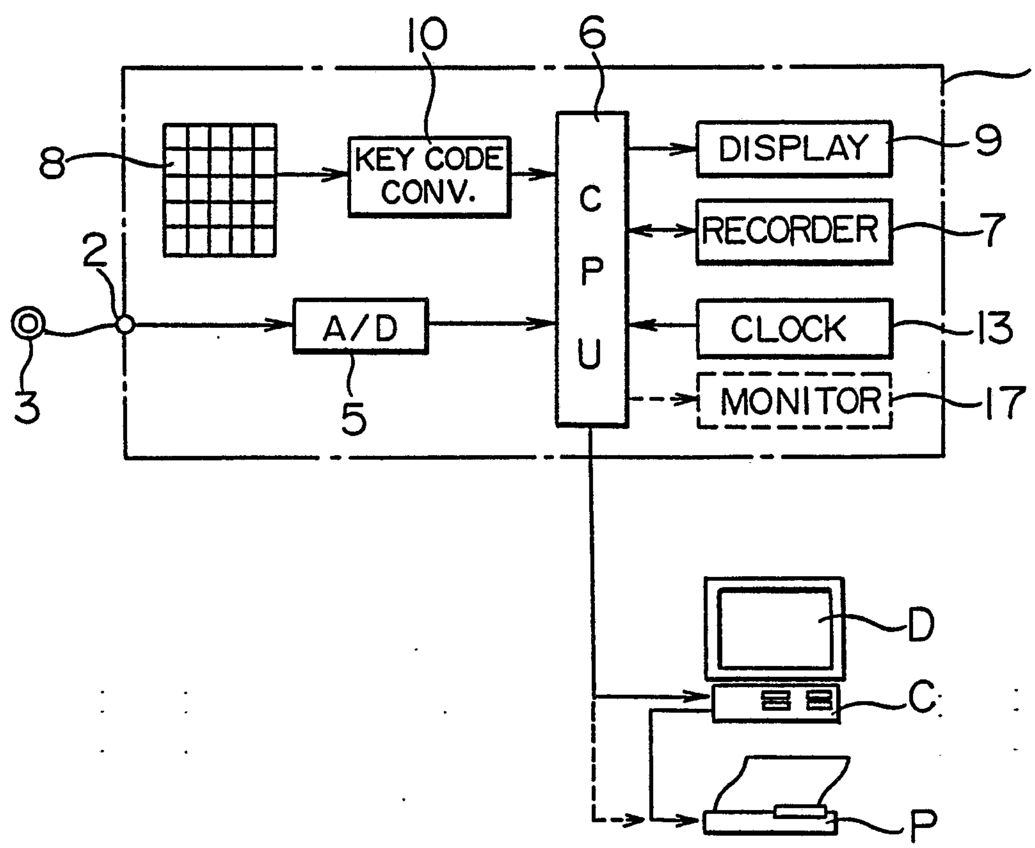
10
6
1
8
KEY CODE CONV.
DISPLAY
9
C P U
RECORDER
7
2
A/D
CLOCK
13
3
5
MONITOR
17
D
C
P

FIG. 3

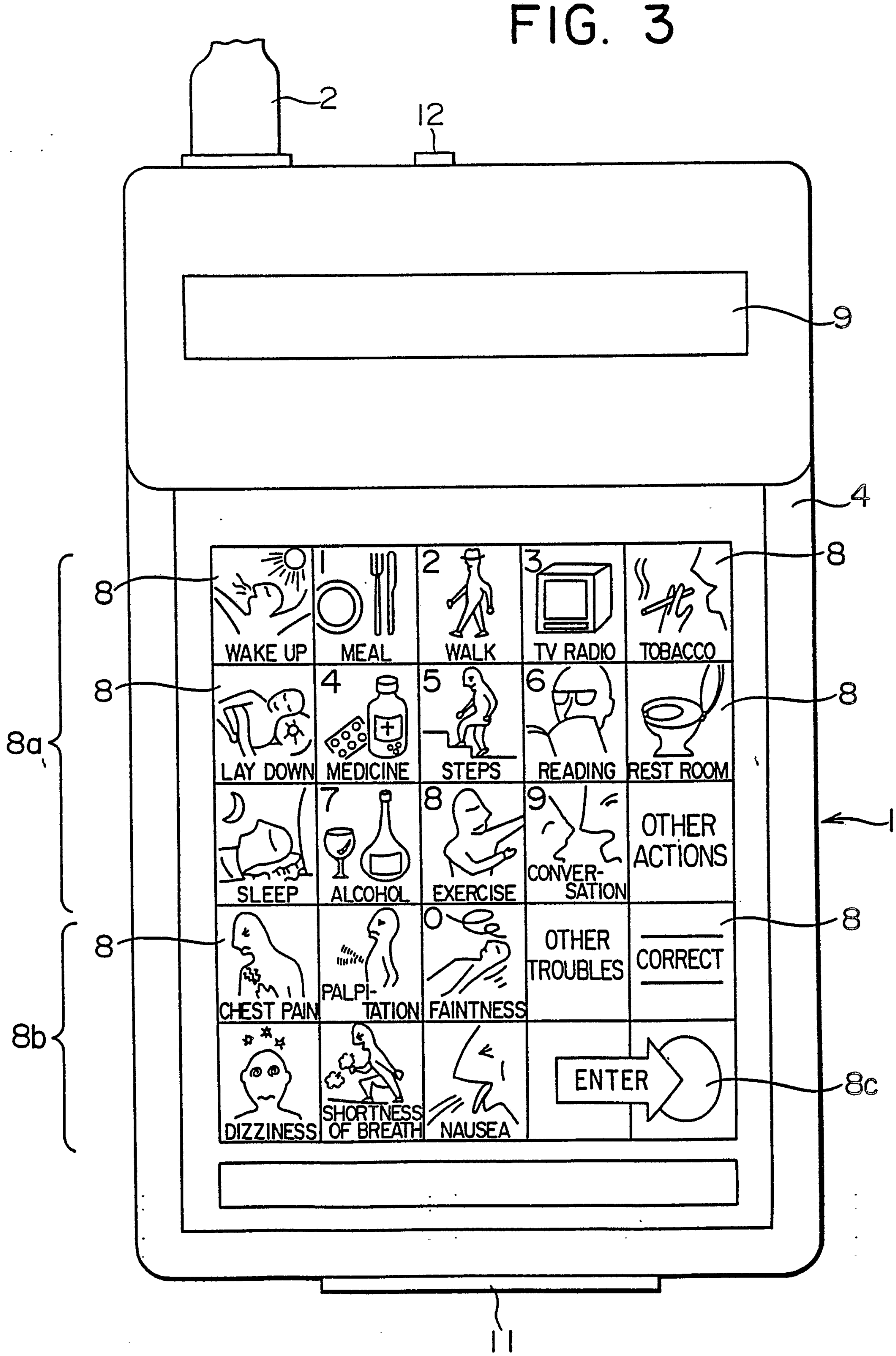
2
12
9
4
8
8a
8b
8c
1
11
WAKE UP
1 MEAL
2 WALK
3 TV RADIO
TOBACCO
LAY DOWN
4 MEDICINE
5 STEPS
6 READING
REST ROOM
SLEEP
7 ALCOHOL
8 EXERCISE
9 CONVER-SATION
OTHER ACTIONS
CHEST PAIN
PALPI-TATION
0 FAINTNESS
OTHER TROUBLES
CORRECT
DIZZINESS
SHORTNESS OF BREATH
NAUSEA
ENTER

FIG. 4

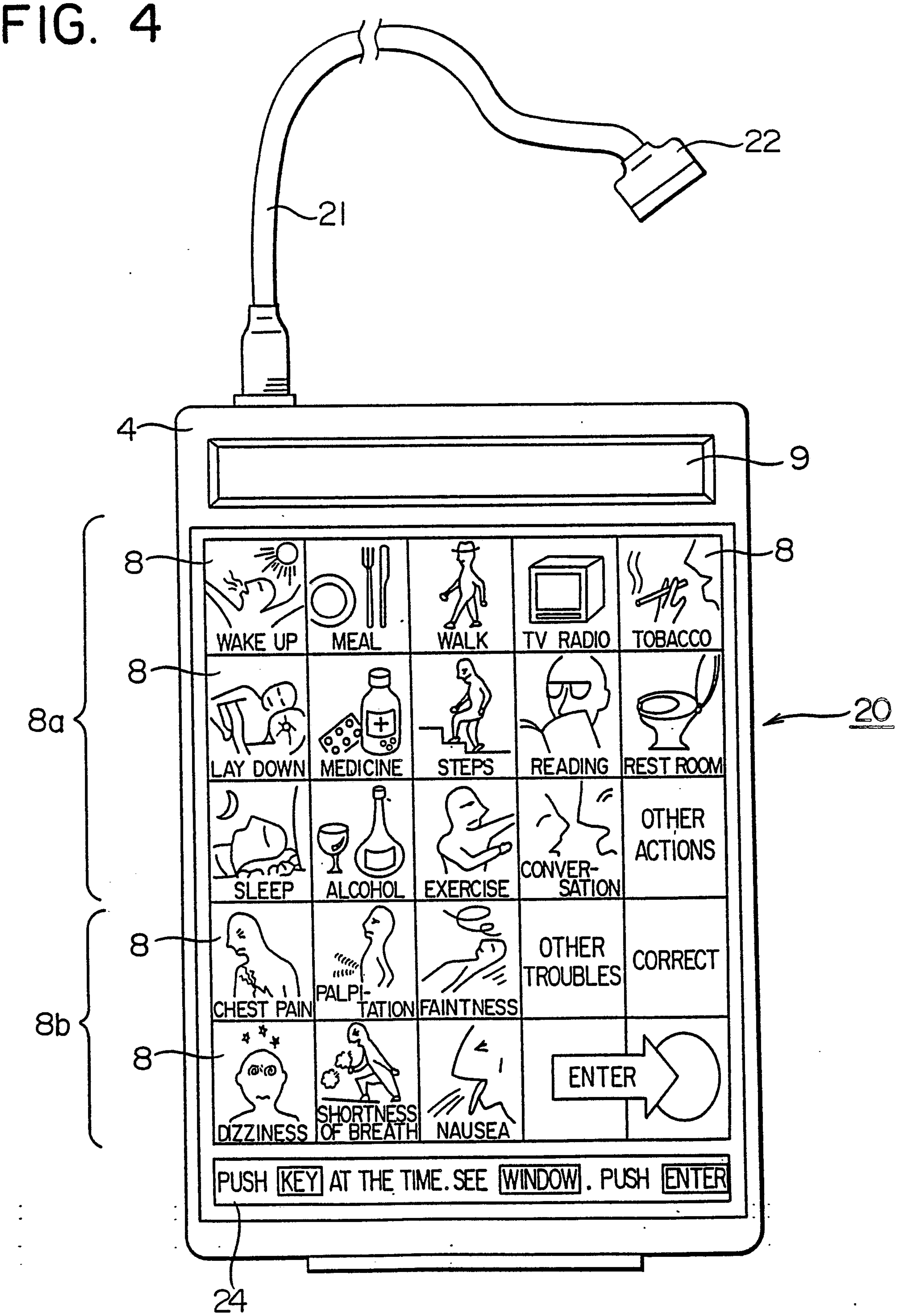
22
21
4
9
8
8
8a
20
8
8
8b
8
WAKE UP
MEAL
WALK
TV RADIO
TOBACCO
LAY DOWN
MEDICINE
STEPS
READING
REST ROOM
SLEEP
ALCOHOL
EXERCISE
CONVER-SATION
OTHER ACTIONS
CHEST PAIN
PALPI-TATION
FAINTNESS
OTHER TROUBLES
CORRECT
DIZZINESS
SHORTNESS OF BREATH
NAUSEA
ENTER
PUSH KEY AT THE TIME. SEE WINDOW. PUSH ENTER
24

FIG. 5

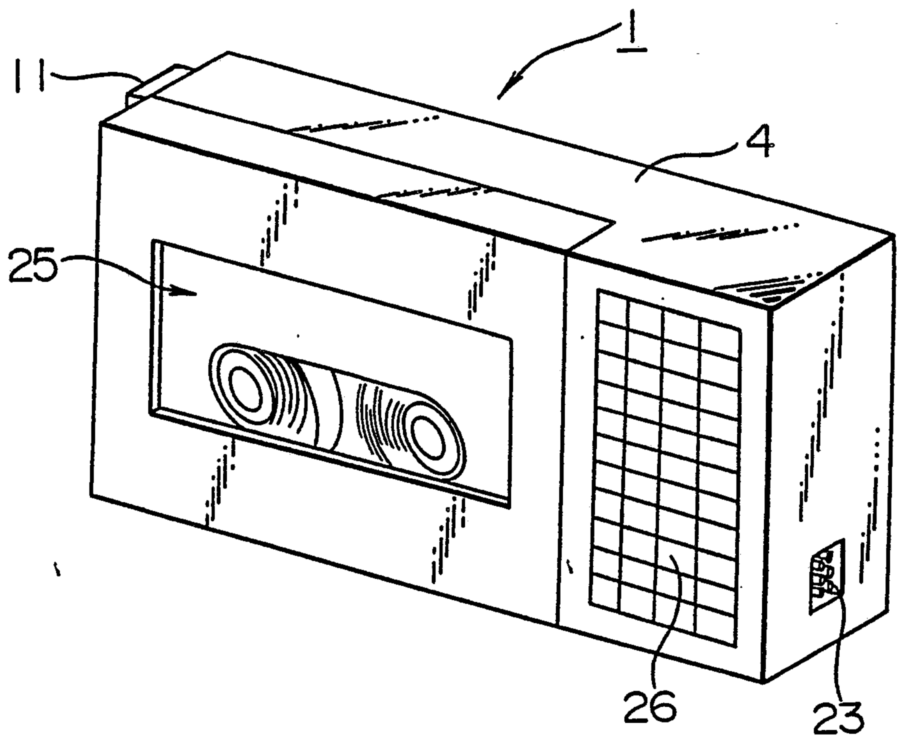

European Patent Office

# EUROPEAN SEARCH REPORT

Application Number

EP 88 30 6136

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 117 330 (M. LUBELL et al.) * Abstract; page 4, lines 16-27; page 5, lines 3-25; page 11, lines 1-21; page 12, lines 10-24; page 13, lines 24-30; page 14, lines 19-22; page 16, line 6 - page 17, lines 9,22-28; figures 1-6 * | 1,2 | A 61 B 5/04 |
| A | | 3,6 | |
| | --- | | |
| Y | US-A-3 779 237 (R.R. GOELTZ et al.) * Abstract; column 5, lines 5-14,67-68; column 6, lines 25-64; column 8, lines 31-38; column 10, lines 11-19; column 12, lines 33-46; column 18, lines 30-43; figures 1-7,14 * | 1,2 | |
| A | | 4,5 | |
| | --- | | |
| A | DE-A-3 539 564 (T. KATAHIRA) * Abstract; page 6, lines 7-26; page 8, lines 16-32; page 9, lines 1-8,20-32; page 11, lines 1-3,18-23; page 12, lines 1-11; figures 1-3 * | 1-3,5,6 | **TECHNICAL FIELDS SEARCHED (Int. Cl.4)** |
| | --- | | |
| A | EP-A-0 101 870 (KONTRON-HOLDING AG) * Abstract; page 3, lines 6-11,25-31; page 4, lines 17-30; page 5, line 34 - page 6, line 8; page 10, claims 1,4,5; figures 1-3 * | 1,3,6 | A 61 B |
| | --- | | |
| A | GB-A-1 331 303 (INTERMEDCRAFT CORP.) * Page 1, lines 39-60; page 4, lines 25-47; figure 1 * | 1,4 | |
| | ----- | | |

The present search report has been drawn up for all claims

| Place of search | Date of completion of the search | Examiner |
|---|---|---|
| THE HAGUE | 13-10-1988 | RIEB K.D. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)